(19) Europäisches Patentamt
European Patent Office
Office européen des brevets

(11) Veröffentlichungsnummer: **0 377 892 B1**

(12) # EUROPÄISCHE PATENTSCHRIFT

(45) Veröffentlichungstag der Patentschrift: **14.09.94**

(51) Int. Cl.[5]: **C07D 237/18**, C07D 413/14, A01N 43/58

(21) Anmeldenummer: **89123882.6**

(22) Anmeldetag: **23.12.89**

Die Akte enthält technische Angaben, die nach dem Eingang der Anmeldung eingereicht wurden und die nicht in dieser Patentschrift enthalten sind.

(54) **3(2H)-Pyridazinonderivate, Verfahren zu ihrer Herstellung und ihre Verwendung zur Bekämpfung von Schädlingen.**

(30) Priorität: **29.12.88 DE 3844227**

(43) Veröffentlichungstag der Anmeldung: **18.07.90 Patentblatt 90/29**

(45) Bekanntmachung des Hinweises auf die Patenterteilung: **14.09.94 Patentblatt 94/37**

(84) Benannte Vertragsstaaten: **BE CH DE ES FR GB IT LI NL**

(56) Entgegenhaltungen:
**EP-A- 0 088 384**
**EP-A- 0 134 439**
**EP-A- 0 199 281**

(73) Patentinhaber: **BASF Aktiengesellschaft**
**Carl-Bosch-Strasse 38**
**D-67063 Ludwigshafen (DE)**

(72) Erfinder: **Leyendecker, Joachim, Dr.**
**Stahlbuehlring 79**
**D-6802 Ladenburg (DE)**
Erfinder: **Theobald, Hans, Dr.**
**Oueichstrasse 6**
**D-6703 Limburgerhof (DE)**
Erfinder: **Kuekenhoehner, Thomas, Dr.**
**Seidelstrasse 2**
**D-6710 Frankenthal (DE)**
Erfinder: **Hofmeister, Peter, Dr.**
**Bernard-Humblot-Strasse 12**
**D-6730 Neustadt (DE)**
Erfinder: **Kuenast, Christoph, Dr.**
**Salierstrasse 2**
**D-6701 Otterstadt (DE)**
Erfinder: **Goetz, Norbert, Dr.**
**Schoefferstrasse 25**
**D-6520 Worms 1 (DE)**

**EP 0 377 892 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 3(2H)-Pyridazinonderivate der allgemeinen Formel I

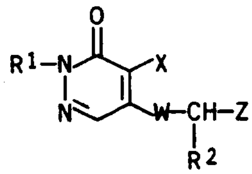

(I),

in der die Substituenten die folgende Bedeutung haben:

- $R^1$ $C_1$-$C_8$-Alkyl,
- $R^2$ Wasserstoff oder $C_1$-$C_4$-Alkyl,
- X Halogen,
- W Sauerstoff oder Schwefel und
- Z einen hetarylsubstituierten Isoxazolylrest

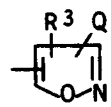

,

wobei

- $R^3$ Wasserstoff, Halogen, $C_1$-$C_8$-Alkyl oder $C_2$-$C_8$-Alkenyl und
- Q Pyrrol-2-yl, Pyrrol-3-yl, Furan-2-yl, Furan-3-yl, Thien-2-yl, Thien-3-yl, Pyrazol-3-yl, Pyrazol-4-yl, Pyrazol-5-yl, Imidazol-2-yl, Imidazol-4-yl, Imidazol-5-yl, 1,2,4-Triazol-3-yl, 1,2,4-Triazol-5-yl, Isoxazol-3-yl, Isoxazol-4-yl, Isoxazol-5-yl, Isothiazol-3-yl, Isothiazol-4-yl, Isothiazol-5-yl, Oxazol-2-yl, Thiazol-2-yl, Thiazol-4-yl, Thiazol-5-yl, 1,3,4-Oxadiazol-5-yl, 1,3,4-Thiadiazol-5-yl, 1,2,4-Oxadiazol-3-yl, 1,2,4-Oxadiazol-5-yl, Pyridin-2-yl, Pyridin-3-yl, Pyridin-4-yl, Pyrimidin-2-yl, Pyrimidin-4-yl, Pyrimidin-5-yl, Pyrazin-2-yl, Pyridazin-3-yl, Pyridazin-4-yl, 1,2,3-Triazin-4-yl, 1,2,3-Triazin-5-yl, 1,2,4-Triazin-3-yl, 1,2,4-Triazin-5-yl, 1,2,4-Triazin-6-yl, 1,3,5-Triazin-2-yl, Tetrahydrofuran-2-yl, Tetrahydrofuran-3-yl, Piperidin-2-yl, Piperidin-3-yl, Piperidin-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, welches unsubstituiert oder ein- bis dreifach mit Halogen, $C_1$-$C_8$-Alkyl, $C_2$-$C_8$-Alkenyl, $C_1$-$C_4$-Halogenalkyl, $C_1$-$C_8$-Alkoxy, $C_2$-$C_8$-Alkoxyalkyl, $C_3$-$C_8$-Cycloalkyl, Cyano und Nitro substituiert ist, bedeuten,

sowie pflanzenverträgliche Salze der 3(2H)-Pyridazinonderivate I.

Außerdem betrifft die Erfindung die Herstellung der Verbindungen I und deren Salze, Schädlingsbekämpfungsmittel, die die Verbindungen I oder deren Salze als Wirkstoffe enthalten sowie ein Verfahren zur Bekämpfung von Schädlingen.

In der älteren deutschen Patentanmeldung P 37 42 266.9 sind 2-tert.-Butyl-5-isoxazolylmethylthio-3(2H)-pyridazinone, die im Isoxazolylrest phenyl- oder arylsubstituiert sind, beschrieben. Aus EP-A-199 281 sind zahlreiche hetarylisch substituierte 3(2H)-Pyridazinone bekannt, die als Hetarylrest aber keinen Isoxazolrest enthalten. Aus EP-A-88 384, EP-A-134 439 und EP-A-183 212 sind weiterhin zahlreiche 5-Benzyl-3(2H)-pyridazinonderivatebekannt. Die insektizide und akarazide Wirkung der vorbeschriebenen Verbindungen ist jedoch nicht immer befriedigend.

Der Erfindung lag daher die Aufgabe zugrunde, neue, speziell substituierte 3(2H)-Pyridazinonderivate mit verbesserter Wirkung bereitzustellen.

Demgemäß wurden die eingangs definierten neuen 3(2H)-Pyridazinonderivate der allgemeinen Formel I, bzw. deren pflanzenverträgliche Salze, sowie ein Verfahren zu ihrer Herstellung gefunden. Ferner wurde gefunden, daß sich die Verbindungen I, oder deren Salze bei sehr guter Pflanzenverträglichkeit hervorragend zur Bekämpfung von Schädlingen eignen.

Die Substituenten in Formel I haben im einzelnen folgende Bedeutungen:

- $R^1$ unverzweigtes oder verzweigtes $C_1$-$C_8$-Alkyl, bevorzugt $C_1$-$C_6$-Alkyl, besonders bevorzugt $C_1$-$6_4$-Alkyl wie Methyl, Ethyl, n-Propyl, i-Propyl, n-Butyl, i-Butyl, s-Butyl und tert.-Butyl,

$R^2$ Wasserstoff,
unverzweigtes oder verzweigtes $C_1$-$C_4$-Alkyl wie für $R^1$ aufgeführt, bevorzugt $C_1$-$C_2$-Alkyl, besonders bevorzugt Methyl,
X Halogen, bevorzugt Chlor oder Brom, besonders bevorzugt Chlor,
W Sauerstoff oder Schwefel, besonders bevorzugt Schwefel,
Z ein ggf. substituierter Isoxazolylrest, in dem
$R^3$ Wasserstoff, bevorzugt Wasserstoff in 4-Position,
Halogen, bevorzugt Fluor, Chlor und Brom, besonders bevorzugt Brom in 5-Position,
unverzweigtes oder verzweigtes $C_1$-$C_8$-Alkyl, bevorzugt unverzweigtes oder verzweigtes $C_1$-$C_4$-Alkyl, besonders bevorzugt Methyl in 5-Position,
unverzweigtes oder verzweigtes $C_2$-$C_6$-Alkenyl, bevorzugt unverzweigtes oder verzweigtes $C_2$-$C_4$-Alkenyl, besonders bevorzugt Vinyl, Methylvinyl und Dimethylvinyl
bedeutet und
Q Pyrrol-2-yl, Pyrrol-3-yl, Furan-2-yl, Furan-3-yl, Thiophen-2-yl, Thiophen-3-yl, Pyrazol-3-yl, Pyrazol-4-yl, Pyrazol-5-yl, Imidazol-2-yl, Imidazol-4-yl, Imidazol-5-yl, 1,2,4-Triazol-3-yl, 1,2,4-Triazol-5-yl, Isoxazol-3-yl, Isoxazol-4-yl, Isoxazol-5-yl, Isothiazol-3-yl, Isothiazol-4-yl, Isothiazol-5-yl, Oxazol-2-yl, Thiazol-2-yl, Thiazol-4-yl, Thiazol-5-yl, 1,3,4-Oxadiazol-2-yl, 1,3,4-Thiadiazol-2-yl, 1,2,4-Oxadiazol-3-yl, 1,2,4-Oxadiazol-5-yl, Pyridin-2-yl, Pyridin-3-yl, Pyridin-4-yl, Pyrimidin-2-yl, Pyrimidin-4-yl, Pyrimidin-5-yl, Pyrazin-2-yl, Pyridazin-3-yl, Pyridazin-4-yl, 1,2,3-Triazin-4-yl, 1,2,3-Triazin-5-yl, 1,2,4-Triazin-3-yl, 1,2,4-Triazin-5-yl, 1,2,4-Triazin-6-yl, 1,3,5-Triazin-2-yl, Tetrahydrofuran-2-yl, Tetrahydrofuran-3-yl, Piperidin-2-yl, Piperidin-3-yl, Piperidin-4-yl, Tetrahydropyran-2-yl,Tetrahydropyran-3-yl, Tetrahydropyran-4-yl, Tetrahydrothiopyran-2-yl, Tetrahydrothiopyran-3-yl, Tetrahydrothiopyran-4-yl, besonders bevorzugt Pyrrol-2-yl, Pyrrol-3-yl, Furan-2-yl, Furan-3-yl, Thiophen-2-yl, Thiophen-3-yl, Imidazol-4-yl, Pyrazol-3-yl, Pyrazol-4-yl, Pyrazol-5-yl, Isoxazol-3-yl, Isoxazol-4-yl, Isoxazol-5-yl, Isothiazol-4-yl, Thiazol-4-yl, Thiazol-5-yl, 1,2,4-Oxadiazol-3-yl, 1,2,4-Oxadiazol-5-yl, 1,3,4-Thiadiazol-2-yl, 1,3,4-Oxadiazol-2-yl, Pyridin-2-yl, Pyridin-3-yl, Pyridin-4-yl, Pyrazin-2-yl, Pyrimidin-4-yl, Pyrimidin-5-yl, Tetrahydropyran-2-yl, Tetrahydropyran-3-yl, Tetrahydropyran-4-yl und Tetrahydrothiopyran-3-yl darstellt
und als Substituenten
Halogen, bevorzugt Fluor, Chlor und Brom, besonders bevorzugt Chlor und Brom,
$C_1$-$C_8$-Alkyl, bevorzugt $C_1$-$C_4$-Alkyl, besonders bevorzugt Methyl, Ethyl, Isopropyl, tert.-Butyl,
$C_2$-$C_8$-Alkenyl, bevorzugt $C_2$-$C_4$-Alkenyl, besonders bevorzugt Ethenyl, 1-Methyl-ethenyl, Propenyl, 2-Methyl-propenyl, $C_1$-$C_4$-Halogenalkyl, bevorzugt mit Fluor oder Chlor substituiertes $C_1$-$C_2$-Halogenalkyl, besonders bevorzugt Trifluormethyl, 2,2,2-Trifluoreth-1-yl,
$C_1$-$C_8$-Alkoxy, bevorzugt $C_1$-$C_3$-Alkoxy, besonders bevorzugt Methoxy, Ethoxy, n-Propyloxy und Isopropyloxy,
$C_2$-$C_8$-Alkoxyalkyl, bevorzugt $C_2$-$C_4$-Alkoxyalkyl, besonders bevorzugt Methoxymethyl, 1-Methoxyethyl, 2-Methoxyethyl, 1-Methoxy-propyl,
$C_3$-$C_8$-Cycloalkyl, bevorzugt $C_3$-$C_5$-Cycloalkyl wie Cyclopropyl, Cyclobutyl und Cyclopentyl, Cyano und Nitro
in Betracht kommen.

Die Verbindungen I sind nach folgender Methode erhältlich:
Die Umsetzung erfolgt zwischen einem 3(2H)-Pyridazinonderivat der allgemeinen Formel II und einem Isoxazol der allgemeinen Formel III in Gegenwart einer Base im Temperaturbereich von (-20) bis 250 °C, vorzugsweise von 20 bis 120 °C nach folgender Reaktionsgleichung:

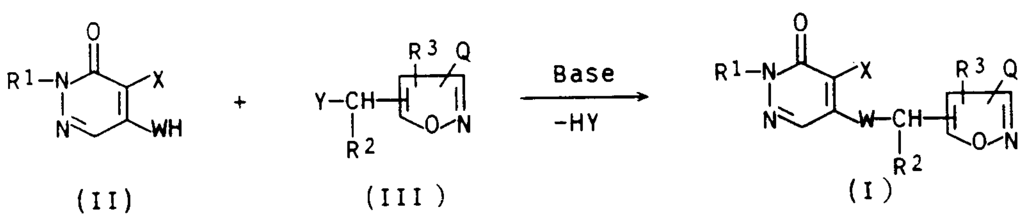


(II) (III) (I)

Die 3(2H)-Pyridazinonderivate der allgemeinen Formel II sind zum Teil in dem belgischen Patent 607 934; in EP-A-134 439, in Angew. Chem. 72, 864ff (1960) und Chem. Pharm. Bull 18, 147ff (1970) beschrieben oder können nach den dort beschriebenen Methoden hergestellt werden.

Die Isoxazole der allgemeinen Formel III sind z. T. aus Bull. Chem. Soc. Jpn. 40, 2604-2607 (1967); WO-A-86/899; WO-A-86/900; J. Het. Chem. 19, 557-560 (1982), J. Chem. Soc. Perkin Trans I. 1976, 570-573 und J. Org. Chem. 26, 1514-1518 (1961) bekannt oder lassen sich nach den in DE-A-22 49 962 und DE-A-27 54 832 beschriebenen Methoden aus Aldoximen durch 1,3-dipolare Cycloaddition mit Propargylalkoholen oder -halogeniden darstellen.

Der Rest Y bedeutet eine Abgangsgruppe wie beispielsweise einen Sulfonsäurerest oder ein Halogen. Unter den Sulfonsäureresten sind Methansulfonyl, Trifluormethansulfonyl, Benzolsulfonyl und p-Toluolsulfonyl, unter den Halogenen sind Chlor und Brom bevorzugt, besonders bevorzugt wird Chlor.

Zur Herstellung der erfindungsgemäßen Verbindungen I nach den vorstehend beschriebenen Methoden setzt man die Ausgangsstoffe üblicherweise in stöchiometrischem Verhältnis ein. Ein überschuß der einen oder anderen Komponente kann von Vorteil sein.

Die Umsetzungen verlaufen gewöhnlich oberhalb von -20°C mit ausreichenden Geschwindigkeiten. 120°C müssen im allgemeinen nicht überschritten werden. Da sie in einigen Fällen unter wärmeentwicklung verlaufen, kann es von Vorteil sein, Kühlmöglichkeiten vorzusehen.

Man setzt normalerweise mindestens äquivalente Mengen einer Base zu II und/oder III zu, kann aber diese auch im überschuß oder gegebenenfalls auch als Lösungsmittel verwenden. Als Basen eignen sich beispielsweise Hydroxide von Alkali- und Erdalkalimetallen, wie Natriumhydroxid, Kaliumhydroxid und Calciumhydroxid, Alkoholate von Alkali- und Erdalkalimetallen, wie Natriummethylat, Natriumethylat, Calciummethanolat oder Kalium-tert.-butylat; Alkali- oder Erdalkalimetallhydride, wie Natriumhydrid, Kaliumhydrid oder Calciumhydrid; Alkali- oder Erdalkalicarbonate, wie Natriumcarbonat, Kaliumcarbonat oder Calciumcarbonat; aliphatische Amine, wie Dimethylamin, Triethylamin oder Diisopropylamin; heterocyclische Amine, wie Piperidin, Piperazin oder Pyrrolidin; aromatische Amine, wie Pyridin oder Pyrrol.

Die Umsetzung wird zweckmäßigerweise in einem Lösungs- oder Verdünnungsmittel durchgeführt. Hierzu eignen sich beispielsweise aliphatische Kohlenwasserstoffe, wie n-Pentan, n-Hexan, das Hexan-Isomerengemisch und Petrolether; halogenierte Kohlenwasserstoffe wie Chlorbenzol, Methylenchlorid, Ethylenchlorid, Chloroform, Tetrachlorethylen; aromatische Kohlenwasserstoffe, wie Benzol, Toluol, die Xylole und deren Isomerengemische, Benzin; Alkohole, wie Methanol, Ethanol, n-Propanol und Isopropanol; Ether, wie Diethyl-, Di-n-butylether, Methyl-tert.-butylether, Tetrahydrofuran und Dioxan; Ketone, wie Aceton, Methylethylketon und Methylisopropylketon; Nitrile, wie Acetonitril und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

Zweckmäßigerweise wird das 3(2H)-Pyridazinon der allgemeinen Formel II in einem Verdünnungs- oder Lösungsmittel vorgelegt, unter anschließender Zugabe des Ausgangsstoffes III. Zur Isolierung der neuen Verbindungen I wird nach üblichen Methoden vorgegangen. Die anfallenden Produkte können durch Umkristallisieren, Extrahieren oder Chromatographieren gereinigt werden.

Zur Darstellung von Salzen, was bei geeigneten hetarylsubstituierten Isoxazolylresten möglich ist, werden die entsprechenden 5-Isoxazolylmethyl-3-(2H)-Pyridazinone I mit üblicherweise verwendeten Salzbildnern, wie z. B. Chlorwasserstoffsäure, Bromwasserstoffsäure, Jodwasserstoffsäure, Schwefelsäure, Phosphorsäure, Ameisensäure, Essigsäure, Oxalsäure, Benzolsulfonsäure, p-Toluolsulfonsäure, Dodecylbenzolsulfonsäure, Methylchlorid, Methylbromid, Methyljodid, Ethylchlorid, Ethylbromid, Ethyljodid, n-Propylchlorid, n-Propylbromid, Dimethylsulfat und Diethylsulfat im Temperaturbereich von 0 bis 150°C, vorzugsweise von 20 bis 120°C umgesetzt.

Die Umsetzung wird zweckmäßigerweise in einem Lösungs- oder Verdünnungsmittel durchgeführt. Hierzu eignen sich beispielsweise aliphatische Kohlenwasserstoffe wie n-Pentan, n-Hexan, das Hexan-Isomerengemisch und Petrolether; aromatische Kohlenwasserstoffe wie Benzol, Toluol, die Xylole und deren Isomerengemische, Benzin; Ether wie Diethylether, Di-n-butylether, Methyl-tert.-butylether, Tetrahydrofuran und Dioxan; Ketone wie Aceton, Methylethylketon und Methylisopropylketon; halogenierte Kohlenwasserstoffe wie Chlorbenzol, Methylenchlorid, Ethylenchlorid, Chloroform und Tetrachlorethylen. Auch Gemische dieser Stoffe können als Lösungsmittel verwendet werden.

Zur Herstellung der Salze geeigneter Verbindungen I nach der vorstehend beschriebenen Methode setzt man die Ausgangsstoffe üblicherweise in stöchiometrischem Verhältnis ein. Ein überschuß der einen oder anderen Komponente kann aber durchaus von Vorteil sein.

Die Umsetzungen verlaufen gewöhnlich oberhalb von 0°C mit ausreichenden Geschwindigkeiten. 120°C müssen im allgemeinen nicht überschritten werden. Da sie in einigen Fällen unter Wärmeentwicklung verlaufen, kann es von Vorteil sein, Kühlmöglichkeiten vorzusehen.

Zur Isolierung der Salze der erfindungsgemäßen Verbindungen I wird nach üblichen Methoden vorgegangen. Die anfallenden Produkte können durch Umkristallisieren, Extrahieren oder Chromatographieren gereinigt werden.

Die 3(2H)-Pyridazinonderivate der Formel I sind geeignet, Schädlinge aus der Klasse der Insekten, Spinnentiere, Nematoden und Schnecken wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Ortiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Geradflügler (Orthoptera) beispielsweise Forficula auricularia, Acheta domestica, Gryllotalpa gryllotalpa, tachycines asynamorus, Locusta migratoria, Stauronotus meroccanus, Schistocerca peregrina, Nomadacris septemfasciata, Melanoplus spretus, Melanoplus femur-rubrum, Blatta orientalis, Blattella germanica, Periplaneta americana, Blabera gigantes.

Zur Klasse der Arachnoidea gehören Spinnentiere (Acarina) beispielsweise Ixodes ficinus, Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schachtii, Heterodera trifolii, Stock- und Blattälchen, z.B. Belonolaimus longicaudatus, Ditylenchus destructor, Ditylenchus dipsaci, Heliocotylenchus multicinctus,

Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Poratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindinngsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 6 werden mit 95 Gew.-Teilen feinteiligem Kaolin innig vermischt. Man erhält auf diese Weise ein Stäubemittel, das 5 Gew.-% des Wirkstoffs enthält.

II. 30 Gew.-Teile der Verbindung Nr. 16 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 43 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,001 und 0,1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 2,0 kg/ha.

Die erfindungsgemäßen Mittel besitzen ebenfalls ausgeprägte molluskizide Eigenschaften sowohl bei Nackt- als auch bei Gehäuseschnecken und eignen sich hervorragend zur Schneckenbekämpfung in landwirtschaftlichen und gärtnerischen Pflanzenkulturen.

Erfindungsgemäß erhält man ein gegen Schnecken wirksames, z. B. als Streumittel formulierbares Präparat durch Verwendung von 3(2H)-Pyridazinonderivaten I in einer wirksamen Menge.

Geeignete Formulierungen sind z.B. in GB 2 087 723 A und EP 190 595 beschrieben. Sie enthalten im allgemeinen einen Fraßstoff, ein Bindemittel, Konservierungsmittel, Farbstoffe, Lockstoffe, Füllmittel, Repellents, Wasser, organische Solventien, oberflächenaktive Stoffe und den Wirkstoff.

Als Fraßstoffe können alle üblichen in derartigen Kodern verwendbaren Futtersubstanzen enthalten sein. Vorzugsweise in Betracht kommen gemahlenes Getreide, wie z.B. Weizenmehl, Weizenschrot und Gerstenschrot, ferner Sojaschrot, Kleie, Reisstärke, Fischmehl, Fleischmehl und Melasse. Es kann entweder nur ein Fraßstoff oder auch ein Gemisch aus mehreren Fraßstoffen vorhanden sein.

Als Bindemittel können alle üblichen für derartige Zwecke verwendbaren Kleber vorhanden sein. Vorzugsweise infrage kommen Methylcellulose, Zucker, Dextrin, Stärke, Alginate, Glycole, Polyvinylpyrrolidon, Lingninsulfonat, Gummiarabicum, Polyvinylalkohol und Polyvinylacetat. Es können ein oder mehrere Bindemittel enthalten sein.

Als Beispiele für gegebenenfalls vorhandene Konservierungsmittel seien 2-Hydroxybiphenyl, Sorbinsäure, p-Hydroxybenzaldehyd, p-Hydroxybenzoesäuremethylester, Benzaldehyd, Benzoesäure, p-Hydroxybenzoesäurepropylester und p-Nitrophenol genannt.

Als Farbstoffe, die als Zusatzstoffe in Betracht kommen, seien anorganische Pigmente, wie Eisenoxid, Titandioxid und Ferrocyanblau und organische Farbstoffe, wie Anthrachinon-, Azo- und Metallphthalocyaninfarbstoffe genannt.

Als Stoffe, die einen Lockreiz auf Bodenschädlinge ausüben, können alle üblichen für diesen Zweck geeigneten Komponenten verwendet werden. Beispielhaft genannt seien Anis und Anisöl.

Als Füllmittel kommen alle üblichen für diesen Zweck verwendbaren Stoffe in Betracht. Vorzugsweise genannt seien Kaoline, Tonerden, Talkum, Kreide und Quarzpulver.

Als Repellents, die eine abweisende Wirkung auf warmblütige Lebewesen wie Hunde und Igel ausüben, können alle üblichen für diesen Zweck geeigneten Komponenten eingesetzt werden. Beispielhaft genannt sei Nonylsäurevanillylamid.

Als organische Solventien kommen alle üblicherweise für die Herstellung von Ködern verwendbaren organischen Lösungsmittel infrage. Vorzugsweise in Betracht kommen niedrig siedende organische Solventien, wie Methanol, Ethanol, Butanol und Methylenchlorid.

Als geeignete oberflächenaktive Stoffe kommen in Betracht nicht-ionische Wirkstoffe, wie Kondensationsprodukte von Polyalkylenoxiden und Alkylphenolen und Fettsäurepolyoxyalkylenestern, z.B. Octylphenoxypolyoxyethanol; kationische Wirkstoffe wie quartäre Ammoniumsalze, z.B. Cetyltrimethylammoniumchlorid oder Cetylpyridiniumchlorid und anionische Wirkstoffe, wie die Natriumsalze von langkettigen Alkylsulfaten, z.B. Natriumlaurylsulfat, Salze von Alkylarylsulfaten, das Natriumsalz der Desoxycholsäure, das Natriumsalz der Taurocholsäure, das Natriumsalz der Tauroglykocholsäure.

Eine andere bevorzugte Anwendungsform ist die Saatgutbeizung mit einer für Beizungen üblichen Formulierung.

Der Wirkstoffgehalt in den einzelnen Anwendungsformen kann in weiten Grenzen variieren, z. B. im Bereich von 0,001 bis 90, insbesondere 0,5 bis 50, vorzugsweise 1 bis 10 Gew.% bei Kornformulierungen und 10 bis 90 Gew.% bei Saatgutbeizen.

Die molluskizide Wirkung der erfindungsgemäßen Mittel erstreckt sich auf landbewohnende und amphibische Schnecken, z. B. solche der Gattungen Deroceras (Agriolimax), Limax, Helix, Helicogona, Cepaea, Milax, Lymnaea (Galba), Achatina, Theba, Cochlicella, Helicarion, Vaginulus. Zu den Schadschnekken gehören z. B. die Nacktschnecken (slugs) Arion ater, A. lusitanicus, A. hortenis, Agriolimax reticulatus, Limax flavus, L. maximus, Milax gagates, Mariella dursumierei, Helicarion salius, Vaginula hedleyi, Pamarion pupillaris sowie die Gehäuseschnecken (snails) Helix aspersa spp., Cepaea nemoralis, Theba pisana, Achatina fulica, A. zanzibarica, Bradybaena spp., Cochlodina spp., Helicella spp., Euomphalia spp.

Formulierungsbeispiel IV

In einem Mischer wurden 2 kg Verbindung Nr. 43, 8 kg Calciumstearat, 0,2 kg Natriumbenzoat, 20 kg Kreide, 0,5 kg blauer Farbstoff und 63,3 kg Weizenkleie gemischt. Diese Mischung wurde anschliepend in einem Kneter mit ausreichend Wasser befeuchtet und geknetet. Danach wurde die feuchte Mischung in einem Extruder zu Schneckenködergranulat mit einem Durchmesser von 3 mm geformt und bei maximal 60 ° C getrocknet.

Formulierungsbeispiel V

Zur Herstellung einer Saatgutbeize wurden gemischt:
480 g Verbindung Nr. 43
20 g handelsübliches Phenolsulfonsäure-Harnstoff-Formaldehyd-Kondensat
40 g Ethylen-Propylen-Blockcopolymerisat mit einem Molgewicht von 10.000
2 g Xanthan-Gummi
0,5 g Rhodamin FB
80 g 1,2-Propylenglykol
5 g Silikon-Antischaummittel
und mit Wasser auf 1 Liter aufgefüllt.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiele

Beispiel 1

2-tert.-Butyl-4-chlor-5-[(3-[2-pyridyl]-isoxazol-5-yl)-methylthio]-3(2H)-pyridazin-3-on (Verbindung Nr. 43)

Zu 7,1 g (0,0325 mol) 2-tert.-Butyl-4-chlor-5-mercapto-3(2H)-pyridazin-3-on und 4,48 g (0,065 mol) Kaliumcarbonat in 40 ml trockenem Dimethylformamid tropft man bei Raumtemperatur (ca. 20 ° C) 6,32 g (0,0325 mol) 5-Chlormethyl-3-(2-pyridyl)-isoxazol in 20 ml trockenem Dimethylformamid. Anschließend rührt man 2 Stunden bei 80 ° C und über Nacht bei Raumtemperatur (ca. 20 ° C) nach. Zur Aufarbeitung gießt man in 200 ml Wasser und extrahiert mit Essigester. Die organische Phase wird über Magnesiumsulfat getrocknet und anschließend das Lösungsmittel im Vakuum verdampft. Der Rückstand wird aus n-Hexan/Essigester = 2/1 umkristallisiert. Man erhält 7,1 g (58 % d. Theorie) 2-tert.-Butyl-4-chlor-5-[(3-[2-pyridyl]-isoxazol-5-yl)-methylthio]-3(2H)-pyridazin-3-on als helles Pulver mit Schmp. 99-102 ° C.

Beispiel 2

2-tert.-Butyl-4-chlor-5-[(3-[3-pyridyl]-isoxazol-5-yl)-methylthio]-3(2H)-pyridazin-3-on (Verbindung Nr. 50)

Zu 8,96 g (0,042 mol) 2-tert.-Butyl-4-chlor-5-mercapto-3(2H)-pyridazin-3-on und 5,66 g (0,082 mol) Kaliumcarbonat in 50 ml trockenem Dimethylformamid tropft man bei Raumtemperatur (ca. 20 ° C) 8 g (0,042 mol) 5-Chlormethyl-3-(3-pyridyl)-isoxazol in 30 ml trockenem Dimethylformamid. Anschließend rührt man über Nacht bei Raumtemperatur (ca. 20 ° C) nach. Zur Aufarbeitung gießt man in 200 ml Wasser und extrahiert mit Essigester. Die organische Phase wird über Magnesiumsulfat getrocknet und anschließend im Vakuum das Lösungsmittel verdampft. Der Rückstand wird aus n-Hexan/Essigester = 4/1 umkristallisiert. Man erhält 3,8 g (25 % d. Theorie) 2-tert.-Butyl-4-chlor-5-[(3-(3-pyridyl)-isoxazol-5-yl)-methylthio]-3(2H)-pyridazin-3-on als helles Pulver mit Schmp. 130-134 ° C.

Beispiel 3

Hydrochlorid der Verbindung 43 (Verbindung Nr. 62)

In eine Lösung von 5 g (0,013 mol) 2-tert.-Butyl-4-chlor-5-[(3-[2-pyridyl]-isoxazol-5-yl)-methylthio]3(2H)-pyridazin-3-on in 100 ml absolutem Diethylether leitet man 30 Minuten trockenes Salzsäuregas ein. Der

ausgefallene weiße Niederschlag wird abgesaugt und getrocknet. Man erhält 4,5 g (85 % der Theorie) des Hydrochlorids der Verbindung Nr. 43 als hygroskopisches Pulver mit Schmp. 167-171 °C.

Entsprechend dieser Herstellvorschriften können die in der folgenden Tabelle 1 beschriebenen Verbindungen und deren Salze hergestellt werden. Die Substitutionsstellen an den Heterocyclen sind jeweils durch einen Strich gekennzeichnet.

Die ohne Angabe physikalischer Kenngrößen in der nachstehenden Tabelle aufgeführten Verbindungen I oder deren Salze können aus den entsprechenden Vorprodukten ohne weiteres erhalten werden und lassen eine gleichartige Wirkung erwarten.

Tabelle : Isoxazolylmethyl-3(2H)-pyridazinonderivate I

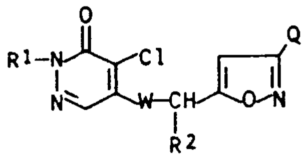

(I)

| Nr. | $R^1$ | $R^2$ | W | Q | phys. Daten Fp. [°C] |
|---|---|---|---|---|---|
| 1 | $CH_3$ | H | O | 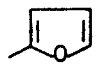 | |
| 2 | $CH_3$ | H | S | 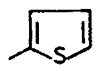 | |
| 3 | $i-C_3H_7$ | H | O | 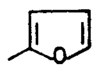 | |
| 4 | $i-C_3H_7$ | H | S | 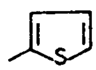 | |
| 5 | $t-C_4H_9$ | H | O | 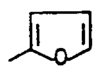 | |
| 6 | $t-C_4H_9$ | H | S | 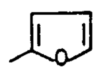 | 109-114 |
| 7 | $CH_3$ | H | S | 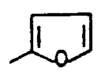 | |
| 8 | $CH_3$ | H | O | 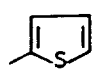 | |
| 9 | $i-C_3H_7$ | H | O | 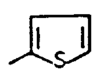 | |
| 10 | $i-C_3H_7$ | H | S | 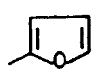 | |
| 11 | $t-C_4H_9$ | H | O | 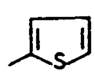 | |
| 12 | $t-C_4H_9$ | H | S | 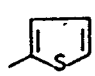 | |

Tabelle : (Fortsetzung)

| Nr. | $R^1$ | $R^2$ | W | Q | phys. Daten Fp. [°C] |
|---|---|---|---|---|---|
| 13 | $t\text{-}C_4H_9$ | $CH_3$ | S | 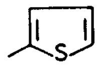 | |
| 14 | $CH_3$ | H | S | 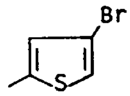 | |
| 15 | $i\text{-}C_3H_7$ | H | S | 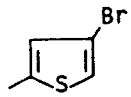 | |
| 16 | $t\text{-}C_4H_9$ | H | S | 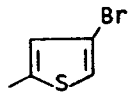 | 110-114 |
| 17 | $t\text{-}C_4H_9$ | $CH_3$ | S | 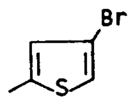 | |
| 18 | $t\text{-}C_4H_9$ | H | S | 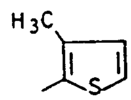 | |
| 19 | $t\text{-}C_4H_9$ | H | S | 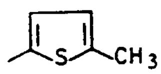 | |
| 20 | $t\text{-}C_4H_9$ | H | S | 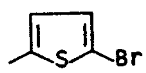 | |
| 21 | $t\text{-}C_4H_9$ | H | S | 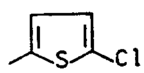 | |
| 22 | $t\text{-}C_4H_9$ | H | S | 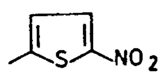 | |
| 23 | $t\text{-}C_4H_9$ | H | S | 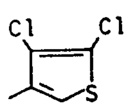 | |
| 24 | $t\text{-}C_4H_9$ | H | S | 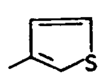 | |

Tabelle : (Fortsetzung)

| Nr. | $R^1$ | $R^2$ | W | Q | phys. Daten Fp. [°C] |
|---|---|---|---|---|---|
| 25 | $t-C_4H_9$ | H | S | 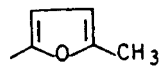 | |
| 26 | $t-C_4H_9$ | H | S | 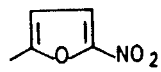 | |
| 27 | $t-C_4H_9$ | H | S | 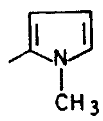 | |
| 28 | $t-C_4H_9$ | H | S | 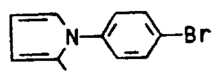 | |
| 29 | $t-C_4H_9$ | H | S | 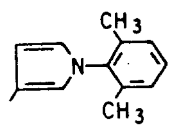 | |
| 30 | $t-C_4H_9$ | H | S | 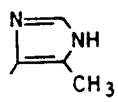 | |
| 31 | $t-C_4H_9$ | H | S | 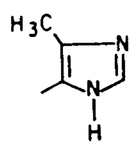 | |
| 32 | $CH_3$ | H | O | 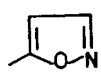 | |
| 33 | $CH_3$ | H | S | 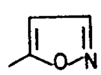 | |
| 34 | $i-C_3H_7$ | H | O | 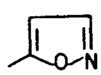 | |
| 35 | $i-C_3H_7$ | H | S | 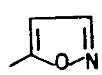 | |
| 36 | $t-C_4H_9$ | H | O | 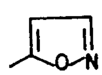 | |

Tabelle : (Fortsetzung)

| Nr. | $R^1$ | $R^2$ | W | Q | phys. Daten Fp. [°C] |
|---|---|---|---|---|---|
| 37 | $t-C_4H_9$ | H | S | 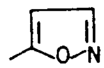 | |
| 38 | $CH_3$ | H | O | 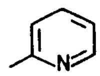 | |
| 39 | $CH_3$ | H | S | 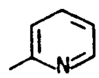 | |
| 40 | $i-C_3H_7$ | H | O | 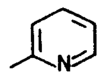 | |
| 41 | $i-C_3H_7$ | H | S | 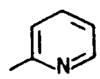 | |
| 42 | $t-C_4H_9$ | H | O | 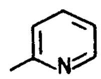 | |
| 43 | $t-C_4H_9$ | H | S | 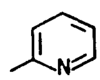 | 99-102 |
| 44 | $t-C_4H_9$ | $CH_3$ | S | 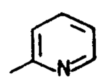 | |
| 45 | $CH_3$ | H | O | 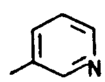 | |
| 46 | $CH_3$ | H | S | 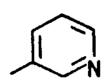 | |
| 47 | $i-C_3H_7$ | H | O | 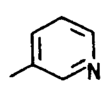 | |
| 48 | $i-C_3H_7$ | H | S | 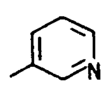 | |
| 49 | $t-C_4H_9$ | H | O | 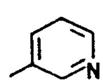 | |

Tabelle : (Fortsetzung)

| Nr. | $R^1$ | $R^2$ | W | Q | phys. Daten Fp. [°C] |
|---|---|---|---|---|---|
| 50 | t-$C_4H_9$ | H | S | 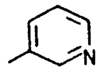 | 130-134 |
| 51 | t-$C_4H_9$ | $CH_3$ | S | 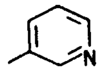 | |
| 52 | t-$C_4H_9$ | $CH_3$ | O | 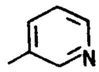 | |
| 53 | $CH_3$ | H | O | 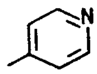 | |
| 54 | $CH_3$ | H | S | 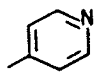 | |
| 55 | i-$C_3H_7$ | H | O | 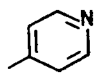 | |
| 56 | i-$C_3H_7$ | H | S | 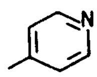 | |
| 57 | t-$C_4H_9$ | H | O | 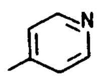 | |
| 58 | t-$C_4H_9$ | H | S | 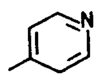 | 141-144 |
| 59 | t-$C_4H_9$ | $CH_3$ | O | 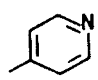 | |
| 60 | t-$C_4H_9$ | $CH_3$ | S | 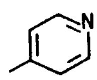 | |
| 61 | t-$C_4H_9$ | H | O | 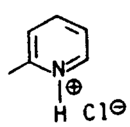 | |
| 62 | t-$C_4H_9$ | H | S | 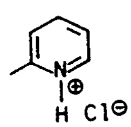 | 167-171 |

Tabelle : (Fortsetzung)

| Nr. | $R^1$ | $R^2$ | W | Q | phys. Daten Fp. [°C] |
|---|---|---|---|---|---|
| 63 | $t-C_4H_9$ | H | O | 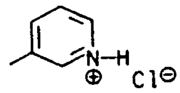 | |
| 64 | $t-C_4H_9$ | H | S | 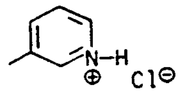 | |
| 65 | $t-C_4H_9$ | H | O | 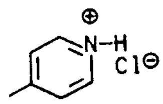 | |
| 66 | $t-C_4H_9$ | H | S | 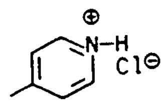 | |
| 67 | $t-C_4H_9$ | H | S | 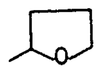 | |
| 68 | $t-C_4H_9$ | $CH_3$ | S | 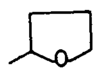 | |
| 69 | $t-C_4H_9$ | H | O | 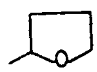 | |
| 70 | $t-C_4H_9$ | $CH_3$ | O | 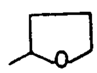 | |
| 71 | $t-C_4H_9$ | H | O | 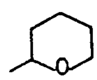 | |
| 72 | $t-C_4H_9$ | $CH_3$ | O | 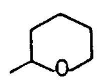 | |
| 73 | $t-C_4H_9$ | H | S | 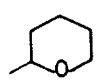 | |
| 74 | $t-C_4H_9$ | $CH_3$ | S | 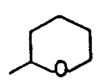 | |

Tabelle : (Fortsetzung)

| Nr. | $R^1$ | $R^2$ | W | Q | phys. Daten Fp. [°C] |
|---|---|---|---|---|---|
| 75 | $CH_3$ | H | S | 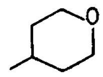 | |
| 76 | $i\text{-}C_3H_7$ | H | S | 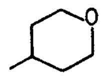 | |
| 77 | $t\text{-}C_4H_9$ | H | S | 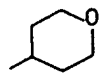 | 125-127 |
| 78 | $t\text{-}C_4H_9$ | $CH_3$ | S | 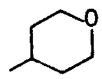 | |
| 79 | $t\text{-}C_4H_9$ | H | S | 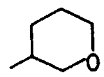 | |
| 80 | $t\text{-}C_4H_9$ | $CH_3$ | S | 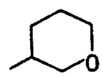 | |
| 81 | $CH_3$ | H | S | 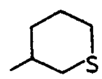 | |
| 82 | $i\text{-}C_3H_7$ | H | S | 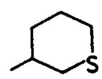 | |
| 83 | $t\text{-}C_4H_9$ | H | S | 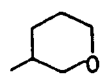 | |
| 84 | $t\text{-}C_4H_9$ | $CH_3$ | S | 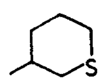 | |
| 85 | $t\text{-}C_4H_9$ | H | O | 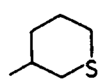 | |
| 86 | $t\text{-}C_4H_9$ | H | S | 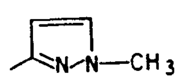 | |
| 87 | $t\text{-}C_4H_9$ | H | S | 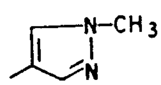 | |

Tabelle : (Fortsetzung)

| Nr. | $R^1$ | $R^2$ | W | Q | phys. Daten Fp. [°C] |
|---|---|---|---|---|---|
| 88 | $t-C_4H_9$ | H | S | 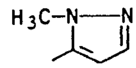 | |
| 89 | $t-C_4H_9$ | H | S | 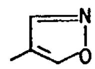 | |
| 90 | $t-C_4H_9$ | H | S | 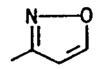 | |
| 91 | $t-C_4H_9$ | H | S | 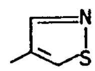 | |
| 92 | $t-C_4H_9$ | H | S | 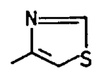 | |
| 93 | $t-C_4H_9$ | H | S | 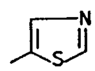 | |
| 94 | $t-C_4H_9$ | H | S | 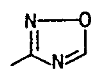 | |
| 95 | $t-C_4H_9$ | H | S | 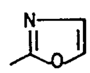 | |
| 96 | $t-C_4H_9$ | H | S | 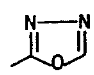 | |
| 97 | $t-C_4H_9$ | H | S | 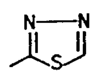 | |
| 98 | $t-C_4H_9$ | H | S | 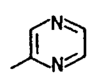 | |
| 99 | $t-C_4H_9$ | H | S | 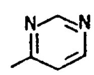 | |
| 100 | $t-C_4H_9$ | H | S | 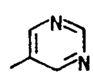 | |
| 189 | $t-C_4H_9$ | H | S | 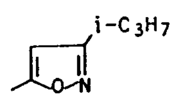 | 93-95 |
| 190 | $t-C_4H_9$ | H | S | 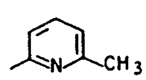 | 126-133 |

Anwendungsbeispiele

In den folgenden Beispielen wurden die Wirkstoffe hinsichtlich ihrer Wirkung auf Tetranychus telarius (Rote Spinne), Aphis fabae (Schwarze Laus) und Dysdercus intermedius (Baumwollwanze) untersucht. Die

Reinheit der Wirkstoffe betrug >95 %.

Als Formulierung wurde ein Emulsionskonzentrat, das 10 Gew.-% Wirkstoff enthält, gewählt. Dieses Emulsionskonzentrat wird durch Zugabe der aktiven Substanz zu einem Gemisch aus 70 Gew.-% Cyclohexanon, 20 Gew.-% Nekanil LN® (≙Lutensol AP6, Netzmittel mit Emulgier- und Dispergierwirkung auf Basis ethoxylierter Alkylphenole) und 10 Gew.-% Emulphor EL® (≙Emulan El®, Emulgator auf Basis ethoxylierter Fettalkohole) erhalten. Die in den Beispielen angegebenen Konzentrationen wurden durch Verdünnen des formulierten Wirkstoffes mit Wasser erhalten.

Beispiel A

Kontaktwirkung auf Aphis fabae (Schwarze Laus), Spritzversuch

Junge Bohnenpflanzen (vicia faba), die mit einer starken Kolonie der Schwarzen Laus befallen sind, werden mit ca. 50 ml der wäßrigen Wirkstoffaufbereitung von allen Seiten bespüht. Nach 24 Stunden erfolgte die Versuchsauswertung in % Mortalität.

In diesem Versuch zeigte die Verbindung des Beispiels 16 eine gute Wirkung.

Beispiel B

Tetranychus telarius (Rote Spinne)

a) Getopfte Buschbohnen, die das zweite Folgeblattpaar zeigen, werden in der Spritzkabine mit der wäßrigen Wirkstoffaufbereitung tropfnaß gespritzt. Die Pflanzen kommen dazu auf einen Drehteller und werden von allen Seiten mit insgesamt 50 ml Spritzbrühe besprüht. Die Pflanzen müssen einen starken Milbenbefall und reichliche Eiablage aufweisen.

Die Wirkung wird nach 5 Tagen mittels Binokular bonitiert.

In diesem Versuch zeigte die Verbindung des Beispiels 43 eine gute Wirkung.

b) Getopfte Buschbohnen, die das zweite Folgeblattpaar zeigen, werden in der Spritzkabine mit der wäßrigen Wirkstoffaufbereitung tropfnaß gespritzt. Die Pflanzen kommen dazu auf einen Drehteller und werden von allen Seiten mit insgesamt 50 ml Spritzbrühe besprüht. Nach 24 Stunden infiziert man die Pflanzen mit Blattstücken, die stark mit Spinnmilben besetzt sind. Die Beurteilung der Wirkung erfolgt nach 12 Tagen.

In diesem Versuch zeigte die Verbindung des Beispiels 43 eine gute Wirkung.

Beispiel C

Ovizide Wirkung auf Dysdercus intermedius (Baumwollwanze)

Stecketiketten werden am oberen Rand mit Klebestreifenstücken (ca. 0,8 cm) beklebt. 24 Stunden vor Versuchsbeginn werden die Eier durch Eintauchen in das "Sammelgefäß" an dem Klebestreifenrand befestigt. Die Eier werden für ca. 5 Sekunden in die wäßrige Wirkstoffaufbereitung getaucht, anschließend läßt man auf Filterpapier abtropfen.

Die so vorbereiteten Etiketten werden mit dem Klebestreifen nach oben in Plastikgefäße gestellt, die mit einer Glasplatte abgedeckt werden. Während des Versuchs wird für ausreichende Feuchtigkeit in den Gefäßen gesorgt, um Austrocknung zu vermeiden. Wenn die Larven im Kontrollversuch geschlüpft sind (nach ca. 8 Tagen) wird bonitiert.

In diesem Versuch zeigte die Verbindung des Beispiels 43 eine gute Wirkung.

**Patentansprüche**

**1.** 3(2H)-Pyridazonderivate der allgemeinen Formel I

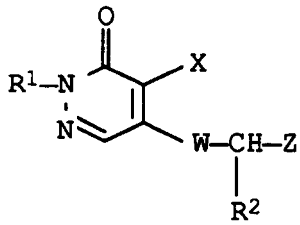

(I),

in der die Substituenten die folgende Bedeutung haben:

- $R^1$ $C_1$-$C_8$-Alkyl;
- $R^2$ Wasserstoff oder $C_1$-$C_4$-Alkyl;
- X Halogen;
- W Sauerstoff oder Schwefel und
- Z einen hetarylsubstituierten Isoxazolylrest

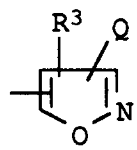

wobei

- $R^3$ Wasserstoff, Halogen, $C_1$-$C_8$-Alkyl oder $C_2$-$C_8$-Alkenyl und
- Q Pyrrol-2-yl, Pyrrol-3-yl, Furan-2-yl, Furan-3-yl, Thien-2-yl, Thien-3-yl, Pyrazol-3-yl, Pyrazol-4-yl, Pyrazol-5-yl, Imidazol-2-yl, Imidazol-4-yl, Imidazol-5-yl, 1,2,4-Triazol-3-yl, 1,2,4-Triazol-5-yl, Isoxazol-3-yl, Isoxazol-4-yl, Isoxazol-5-yl, Isothiazol-3-yl, Isothiazol-4-yl, Isothiazol-5-yl, Oxazol-2-yl, Thiazol-2-yl, Thiazol-4-yl, Thiazol-5-yl, 1,3,4-Oxadiazol-5-yl, 1,3,4-Thiadiazol-5-yl, 1,2,4-Oxadiazol-3-yl, 1,2,4-Oxadiazol-5-yl, Pyridin-2-yl, Pyridin-3-yl, Pyridin-4-yl, Pyrimidin-2-yl, Pyrimidin-4-yl, Pyrimidin-5-yl, Pyrazin-2-yl, Pyridazin-3-yl, Pyridazin-4-yl, 1,2,3-Triazin-4-yl, 1,2,3-Triazin-5-yl, 1,2,4-Triazin-3-yl, 1,2,4-Triazin-5-yl, 1,2,4-Triazin-6-yl, 1,3,5-Triazin-2-yl, Tetrahydrofuran-2-yl, Tetrahydrofuran-3-yl, Piperidin-2-yl, Piperidin-3-yl, Piperidin-4-yl, Tetrahydropyran-2-yl,Tetrahydropyran-3-yl, Tetrahydropyran-4-yl, Tetrahydrothiopyran-2-yl, Tetrahydrothiopyran-3-yl, Tetrahydrothiopyran-4-yl, welches unsubstituiert oder ein- bis dreifach mit Halogen, $C_1$-$C_8$-Alkyl, $C_2$-$C_8$-Alkenyl, $C_1$-$C_4$-Halogenalkyl, $C_1$-$C_8$-Alkoxy, $C_2$-$C_8$-Alkoxyalkyl, $C_3$-$C_8$-Cycloalkyl, Cyano und Nitro substituiert ist, bedeuten,

sowie pflanzenverträgliche Salze der 3(2H)-Pyridazinonderivate I.

**2.** 3(2H)-Pyridazonderivate der allgemeinen Formel I gemäß Anspruch 1, in denen

- $R^2$ Wasserstoff oder Methyl;
- X Chlor und
- W Schwefel bedeutet,

sowie pflanzenverträgliche Salze der 3(2H)-Pyridazinonderivate I.

**3.** Verfahren zur Herstellung von 3(2H)-Pyridazinonderivaten der Formel I gemäß Anspruch 1, dadurch gekennzeichnet, daß man in an sich bekannter Weise 3(2H)-Pyridazinone der allgemeinen Formel II

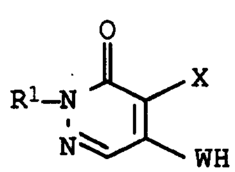

(II),

mit einer Verbindung der allgemeinen Formel III

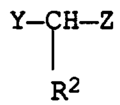

(III),

in der $R^2$ und Z die obengenannten Bedeutungen haben und Y für eine Abgangsgruppe steht, in Gegenwart einer Base umsetzt.

4. Verfahren zur Herstellung von Salzen der 3-(2H)Pyridazinonderivate I, dadurch gekennzeichnet, daß man in an sich bekannter Weise Verbindungen der allgemeinen Formel I mit üblicherweise verwendeten Salzbildnern umsetzt.

5. Schädlingsbekämpfungsmittel, enthaltend ein 3(2H)-Pyridazinonderivat I gemäß den Ansprüchen 1 und 2 oder ggf. dessen Salze neben hierfür üblichen Trägerstoffen.

6. Schädlingsbekämpfungsmittel nach Anspruch 5, dadurch gekennzeichnet, daß es 0,1 bis 95 Gew.-% eines 3(2H)-Pyridazinonderivates I enthält.

7. Verwendung von 3(2H)-Pyridazinonderivaten I gemäß den Ansprüchen 1 oder 2 oder ggf. deren Salzen zur Bekämpfung von Schädlingen.

8. Verwendung von 3(2H)-Pyridazinonderivaten I gemäß den Ansprüchen 1 oder 2 oder ggf. deren Salzen zur Herstellung von Schneckenbekämpfungsmitteln.

9. Verfahren zur Herstellung molluskizider Mittel, dadurch gekennzeichnet, daß man 3(2H)-Pyridazinonderivate I mit inerten Verdünnungsmitteln und/oder oberflächenaktiven Stoffen vermischt.

10. Schneckenfraßköder, gekennzeichnet durch den Gehalt an 3(2H)-Pyridazinonderivaten I.

11. Saatgutbeizmittel, gekennzeichnet durch den Gehalt an 3(2H)-Pyridazinonderivaten I.

12. Verfahren zur Bekämpfung von Schädlingen, dadurch gekennzeichnet, daß man die Schädlinge und/oder Räume mit einer für Schädlinge wirksamen Menge eines 3(2H)-Pyridazinonderivates der allgemeinen Formel I gemäß Anspruch 1 oder ggf. dessen pflanzenverträgliche Salze behandelt.

**Claims**

1. A 3(2H)-pyridazinone derivative of the general formula I

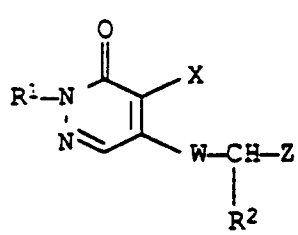

(I)

where
$R^1$ is $C_1$-$C_8$-alkyl,
$R^2$ is hydrogen or $C_1$-$C_4$-alkyl,
X is halogen,
W is oxygen or sulfur and
Z is a hetaryl-substituted isoxazolyl radical

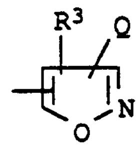

where
$R^3$ is hydrogen, halogen, $C_1$-$C_8$-alkyl or $C_2$-$C_8$-alkenyl and Q is pyrrol-2-yl, pyrrol-3-yl, furan-2-yl, furan-3-yl, thien-2-yl, thien-3-yl, pyrazol-3-yl, pyrazol-4-yl, pyrazol-5-yl, imidazol-2-yl, imidazol-4-yl, imidazol-5-yl, 1,2,4-triazol-3-yl, 1,2,4-triazol-5-yl, isoxazol-3-yl, isoxazol-4-yl, isoxazol-5-yl, isothiazol-3-yl, isothiazol-4-yl, isothiazol-5-yl, oxazol-2-yl, thiazol-2-yl, thiazol-4-yl, thiazol-5-yl, 1,3,4-oxadiazol-5-yl, 1,3,4-thiadiazol-5-yl, 1,2,4-oxadiazol-3-yl, 1,2,4-oxadiazol-5-yl, pyrid-2-yl, pyrid-3-yl, pyrid-4-yl, pyrimidin-2-yl, pyrimidin-4-yl, pyrimidin-5-yl, pyrazin-2-yl, pyridazin-3-yl, pyridazin-4-yl, 1,2,3-triazin-4-yl, 1,2,3-triazin-5-yl, 1,2,4-triazin-3-yl, 1,2,4-triazin-5-yl, 1,2,4-triazin-6-yl, 1,3,5-triazin-2-yl, tetrahydrofuran-2-yl, tetrahydrofuran-3-yl, piperidin-2-yl, piperidin-3-yl, piperidin-4-yl, tetrahydropyran-2-yl, tetrahydropyran-3-yl, tetrahydropyran-4-yl, tetrahydrothiopyran-2-yl, tetrahydrothiopyran-3-yl or tetrahydrothiopyran-4-yl, which is unsubstituted or monosubstituted to trisubstituted by halogen, $C_1$-$C_8$-alkyl, $C_2$-$C_8$-alkenyl, $C_1$-$C_4$-haloalkyl, $C_1$-$C_8$-alkoxy, $C_2$-$C_8$-alkoxyalkyl, $C_3$-$C_8$-cycloalkyl, cyano or nitro, and plant-tolerated salts of the 3(2H)-pyridazinone derivative I.

2. A 3(2H)-pyridazinone derivative of the general formula I as claimed in claim 1, in which
$R^2$ is hydrogen or methyl,
X is chlorine and
W is sulfur,
and plant-tolerated salts of the 3(2H)-pyridazinone derivative I.

3. A process for the preparation of a 3(2H)-pyridazinone derivative of the formula I as claimed in claim 1, wherein a 3(2H)-pyridazinone of the general formula II

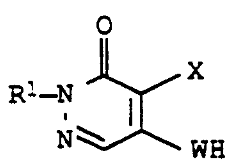

(II)

is reacted in a conventional manner with a compound of the general formula III

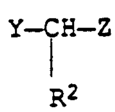

(III)

where $R^2$ and Z have the abovementioned meanings and Y is a leaving group, in the presence of a base.

4. A process for the preparation of a salt of a 3-(2H)-pyridazinone derivative I, wherein a compound of the general formula I is reacted with a customarily used salt former in a conventional manner.

5. A pesticide containing a 3(2H)-pyridazinone derivative I as claimed in claims 1 and 2 or a salt thereof, in addition to conventional carriers.

6. A pesticide as claimed in claim 5, which contains from 0.1 to 95% by weight of a 3(2H)-pyridazinone derivative I.

7. Use of a 3(2H)-pyridazinone derivative I as claimed in claim 1 or 2 or a salt thereof for controlling pests.

8. Use of a 3(2H)-pyridazinone derivative I as claimed in claim 1 or 2 or a salt thereof for the preparation of molluscicides.

9. A process for the preparation of molluscicides, wherein a 3(2H)-pyridazinone derivative I is mixed with inert diluents and/or surfactants.

10. A snail or slug bait, which contains a 3(2H)-pyridazinone derivative I.

11. A seed dressing, which contains a 3(2H)-pyridazinone derivative I.

12. A method for controlling pests, wherein the pests and/or spaces are treated with an amount, effective for pests, of a 3(2H)-pyridazinone derivative of the general formula I as claimed in claim 1 or, if required, a plant-tolerated salt thereof.

## Revendications

1. Dérivés de 3(2H)-pyridazone de la formule générale I

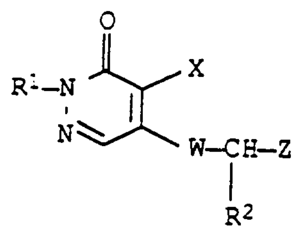

(I),

dans laquelle des substituants ont les significations suivantes :
$R^1$, alkyle en C1-C8;
$R^2$, hydrogène ou alkyle en C1-C4;
X, halogène;
W, oxygène ou soufre et
Z, un reste isoxazolyle substitué par hétaryle

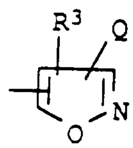

$R^3$ représentant hydrogène, halogène, alkyle en C1-C8 ou alcényle en C2-C8 et
Q pyrrol-2-yle, pyrrol-3-yle, furan-2-yle, furan-3-yle, thien,-2-yle, thien-3-yle, pyrazol-3-yle, pyrazol-4-yle, pyrazol-5-yle, imidazol-2-yle, imidazol-4-yle, imidazol-5-yle, 1,2,4-triazol-3-yle, 1,2,4-triazol-5-yle, isoxazol-3-yle, isoxazol-4-yle, isoxazol-5-yle, isothiazol-3-yle, isothiazol-4-yle, isothiazol-5-yle, oxazol-2-yle, thiazol-2-yle, thiazol-4-yle, thiazol-5-yle, 1,3,4-oxadiazol-5-yle, 1,3,4-thiadiazol-5-yle, 1,2,4-oxadiazol-3-yle, 1,2,4-oxadiazol-5-yle, pyridin-2-yle, pyridin-3-yle, pyridin-4-yle, pyrimidin-2-yle, pyrimidin-4-yle, pyrimidin-5-yle, pyrazin-2-yle, pyridazin-3-yle, pyridazin-4-yle, 1,2,3-triazin-4-yle, 1,2,3-triazin-5-yle, 1,2,4-triazin-3-yle, 1,2,4-triazin-5-yle, 1,2,4-triazin-6-yle, 1,3,5-triazin-2-yle, tétrahydrofuran-2-yle, tétrahydrofuran-3-yle, pipéridin-2-yle, pipéridin-3-yle, pipéridin-4-yle, tétrahydropyran-2-yle, tétrahydropyran-3-yle, tétrahydrothiopyran-4-yle, tétrahydrothiopyran-2-yle, tétrahydrothiopyran-3-yle, tétrahydrothiopyran-4-yle qui n'est pas substitué ou est substitué une à trois fois par halogène, alkyle en C1-C8, alcényle en C2-C8 halogénoalkyle en C1-C4 alcoxy en C1-C8, alcoxyalkyle en C2-C8, cycloalkyle en C3-C8 cyano et nitro
ainsi que les sels des dérivés de 3(2H)pyridazone I acceptables pour les plantes.

2. Dérivés de 3(2H)-pyridazone de la formule I selon la revendication 1, dans laquelle
$R^2$ représente hydrogène ou méthyle

X, chlore et
W, soufre
ainsi que les sels des dérivés de 3(2H)-pyridazone I acceptables pour les plantes.

**3.** Procédé de préparation de dérivés de 3(2H)-pyridazone de la formule I selon la revendication 1 caractérisé par le fait que l'on fait réagir, en présence d'une base d'une manière connue en soi, une 3-(2H)-pyridazinone de la formule générale II

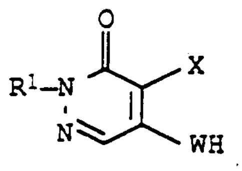

(II),

avec un composé de la formule générale III

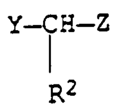

(III),

dans laquelle $R^2$ et Z ont les significations sus-indiqués et y est mis pour un groupe éliminable.

**4.** Procédé pour la préparation de sels des dérivés de 3(2H)-pyridazinone I, caractérisé par le fait qu'on fait réagir de manière connue en soi des composés de la formule générale I avec des formateurs de sels usuellement employés.

**5.** Agent pesticide, contenant un dérivé de 3(2H)-pyridazinone I selon la revendication 1 et 2 ou éventuellement ses sels à côté de véhicules usuels pour cela.

**6.** Agent pesticide selon la revendication 5, caractérisé par le fait qu'il contient 0,1 à 95 % en poids d'un dérivé de 3(2H)-pyridazinone I.

**7.** Utilisation de dérivés de 3(2H)-pyridazinone I selon les revendications 1 ou 2 ou éventuellement leurs sels pour lutter contre les parasites.

**8.** Utilisation de dérivés de 3(2H)-pyridazinone I selon les revendications 1 ou 2 ou éventuellement leurs sels pour préparer des agents de lutte contre les limaces.

**9.** Procédé de préparation d'agent mollusquicide, caractérisé par le fait que l'on mélange des dérivés de 3(2H)-pyridazinone I avec des diluants inertes et/ou des matières tensio-actives.

**10.** Appat d'injection par limaces, caractérisé par la teneur en dérivés de 3(2H)-pyridazinone I.

**11.** Agent de désinfection des semences, caractérisé par la teneur en dérivés de 3(2H)-pyridazinone I.

**12.** Procédé pour lutter contre des parasites, caractérisé par le fait que l'on traite les parasites et/ou des espaces avec une quantité efficace contre les parasites d'un dérivé de 3(2H)-pyridazinone de la formule I selon la revendication 1 ou éventuellement leurs sels acceptables pour les plantes.